# EUROPEAN PATENT SPECIFICATION

(11) **EP 1 179 076 B1**
(45) Date of publication and mention of the grant of the patent: **06.04.2005**
(21) Application number: 00951336.7
(22) Date of filing: 05.07.2000
(51) Int. Cl.: C12N 15/77, C12N 15/53, C12N 9/02, C12P 13/08

(54) **PROCESS FOR THE FERMENTATIVE PREPARATION OF L-AMINO ACIDS WITH AMPLIFICATION OF THE GND GENE**
VERFAHREN ZUR FERMENTATIVEN HERSTELLUNG VON L-AMINOSÄUREN UNTER VERWENDUNG EINES AMPLIFIZIERTEN GND-GENS
PROCEDE DE PREPARATION PAR FERMENTATION DE L-AMINOACIDES AVEC AMPLIFICATION DU GENE GND

(30) Priority: 20.03.2000 US 531265
(43) Date of publication of application: 13.02.2002
(73) Proprietor: Degussa AG, 40474 Düsseldorf (DE); National University of Ireland, Galway (IE)
(72) Inventor: DUNICAU, L., K., (IE); MCCORMACK, Ashling, Athlone, County Westmeath (IE); STAPELTON, Cliona, Roscrea, County Tipparary (IE); BURKE, Kevin, Galway, County Galway (IE); MÖCKEL, Bettina, D-40597 Düsseldorf (DE)
(86) International application number: PCT/EP2000/006299
(87) International publication number: WO 2001/071012

(56) References cited:
- EP-A- 0 358 940
- EP-A- 0 435 132
- EP-A- 0 472 869
- EP-A- 0 733 712
- WO-A-01/04322
- WO-A-01/07626
- US-A- 5 158 891
- PATENT ABSTRACTS OF JAPAN vol. 1998, no. 01, 30 January 1998 (1998-01-30) & JP 09 224662 A (MITSUBISHI CHEM CORP), 2 September 1997 (1997-09-02) cited in the application
- SONNEN H ET AL.: "Characterization of pGA1, a new plasmid from Corynebacterium glutamicum LP-6" GENE, vol. 107, no. 1, 1991, pages 69-74, XP002169154
- VALLINO J J ET AL: "Carbon flux distribution at the glucose-6-phosphate branch point in Corynebacterium glutamicum during lysine overproduction" BIOTECHNOLOGY PROGRESS, vol. 10, 1994, pages 327-334, XP000960466 ISSN: 8756-7938
- SUGIMOTO S-I ET AL.: "Regulation of 6-phosphogluconate dehydrogenase in Brevibacterium flavum" AGRIC. BIOL. CHEM., vol. 51, no. 5, 1987, pages 1257-1263, XP002169156
- DATABASE EMBL [Online] EMBL; ID ECPOXB, AC X04105, 9 March 1987 (1987-03-09) GRABAU C ET AL.: "E. coli poxB gene for pyruvate oxidase" XP002161531
- KRÄMER R: "Genetic and physiological approaches for the production of amino acids" JOURNAL OF BIOTECHNOLOGY, vol. 45, no. 1, 12 February 1996 (1996-02-12), pages 1-21, XP004036833
- NESVERA J ET AL.: "Plasmid pGA1 from Corynebacterium glutamicum codes for a gene product that positively influences plasmid copy number" JOURNAL OF BACTERIOLOGY, vol. 179, no. 5, March 1997 (1997-03), pages 1525-1532, XP002169155 cited in the application
- EIKMANNS B J ET AL.: "A family of Corynebacterium glutamicum/Escherichia coli shuttle vectors for cloning, controlled gene expression, and promoter probing" GENE, vol. 102, 1991, pages 93-98, XP002153371
- MORITZ B ET AL.: "Kinetic properties of the glucose-6-phosphate and 6-phosphogluconate dehydrogenases from Corynebacterium glutamicum and their application for predicting pentose phosphate pathway flux in vivo." EUROPEAN JOURNAL OF BIOCHEMISTRY, vol. 267, no. 12, June 2000 (2000-06), pages 3442-3452, XP000982366

## Description

The invention relates to a process for the fermentative preparation of L-amino acids, in particular L-lysine, L-threonine, L-isoleucine and L-tryptophan, using coryneform bacteria in which at least the gnd gene is amplified.

### Prior art

L-Amino acids are used in animal nutrition, in human medicine and in the pharmaceuticals industry.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acids such as e.g. threonine are obtained in this manner.

Methods of the recombinant DNA technique have also been employed for some years for improving the strain of Corynebacterium glutamicum strains which produce L-amino acids.

WO 01/07626 describes the production of L-amino acids, comprising culturing an altered bacterial cell (I), having an increased amount of NADPH (reduced nicotinamide adenine dinucleotide phosphate) or a decreased amount of 6-phosphoglucose isomerase (Pgi) enzymatic activity, as compared to an unaltered bacterial cell, where yields of L-amino acid from (I) are greater than yields from unaltered bacterial cell.

In EP-A-733712 the productivity of the production of substances such as L-amino-acids, antibiotics, vitamins, growth factors, and physiologically active substances by microbial fermentation is improved by improving the production of reduced nicotinamide adenine dinucleotide phosphate (NADPH) in the microbial cells. This may be carried out by transforming the cells to improve the enzyme activity of nicotinamide nucleotide transhydrogenase (NNT) in the microbial cells, to improve the expression of the NNT gene, and improve the copy number of the NNT gene in the cells. Suitable microorganisms include strains of Escherichia coli, Corynebacterium and Brevibacterium . USE - The method may be used for more efficient large-scale production of substances for use in the pharmaceutical, food, animal feedstuff and chemical industries, esp. L-amino acids, antibiotics, vitamins, growth factors and other physiologically active substances

### Object of the invention

The inventors had the object of providing improved processes for the fermentative preparation of L-amino acids with coryneform bacteria.

### Description of the invention

L-Amino acids are used in human medicine and in the pharmaceuticals industry, in the foodstuffs industry and especially in animal nutrition. There is therefore a general interest in providing new improved processes for the preparation of amino acids.

The invention provides a process for the fermentative preparation of L-amino acids, in particular L-lysine, L-threonine, L-isoleucine and L-tryptophan, using coryneform bacteria in which the nucleotide sequence which codes for the enzyme 6-phosphogluconate dehydrogenase (EC number 1.1.1.44) (gnd gene) is amplified, in particular over-expressed.

Specifically, the invention provides a process for the preparation of L-amino acids by fermentation of coryneform bacteria which comprises carrying out the following steps:
a) fermentation of the desired L-amino acid-producing bacteria in which at least the gnd gene is amplified and at least the poxB gene is attenuated,
b) concentration of the L-amino acid in the medium or in the cells of the bacteria and
c) isolation of the L-amino acid produced.

The strains employed preferably already produce L-amino acids before amplification of the gnd gene.

Preferred embodiments are to be found in the claims.

The term "amplification" in this connection describes the increase in the intracellular activity of one or more enzymes in a microorganism which are coded by the corresponding DNA, for example by increasing the number of copies of the gene or genes, using a potent promoter or using a gene which codes for a corresponding enzyme having a high activity, and optionally combining these measures.

The microorganisms which the present invention provides can prepare L-amino acids from glucose, sucrose, lactose, fructose, maltose, molasses, starch, cellulose or from glycerol and ethanol. They are representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum, are, for example, the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Brevibacterium flavum ATCC14067
Brevibacterium lactofermentum ATCC13869
Brevibacterium divaricatum ATCC14020
and L-amino acid-producing mutants prepared therefrom,
such as, for example, the L-threonine-producing strains
Corynebacterium glutamicum ATCC21649
Brevibacterium flavum BB69
Brevibacterium flavum DSM5399
Brevibacterium lactofermentum FERM-BP 269
Brevibacterium lactofermentum TBB-10
and such as, for example, the L-isoleucine-producing strains
Corynebacterium glutamicum ATCC 14309
Corynebacterium glutamicum ATCC 14310
Corynebacterium glutamicum ATCC 14311
Corynebacterium glutamicum ATCC 15168
Corynebacterium ammoniagenes ATCC 6871
and such as, for example, the L-tryptophan-producing strains
Corynebacterium glutamicum ATCC21850
Corynebacterium glutamicum KY9218(pKW9901)
and such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464
Corynebacterium glutamicum DSM5715
Corynebacterium glutamicum DM58-1
Corynebacterium glutamicum DSM12866.

It has been found that coryneform bacteria produce L-amino acids, in particular L-lysine, L-threonine, L-isoleucine and L-tryptophan, in an improved manner after over-expression of the gnd gene which codes for 6-phosphogluconate dehydrogenase (EC number 1.1.1.44) and attenuation of the poxB gene which codes for the pyruvate oxidase.

The gnd gene codes for the enzyme 6-phosphogluconate dehydrogenase , which catalyses the oxidative decarboxylation of 6-phosphogluconic acid to ribulose 5-phosphate. The nucleotide sequence of the gnd gene is disclosed in JP-A-9-224662. The gnd gene described in the text reference mentioned is used according to the invention for the first time. Alleles of the gnd gene which result present here in plasmids with a varying number of copies, or are integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification EPS 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15-24 (1993)), in Japanese Laid-Open Specification JP-A-10-229891, in Jensen and Hammer (Biotechnology and Bioengineering 58, 191-195 (1998)) and in known textbooks of genetics and molecular biology.

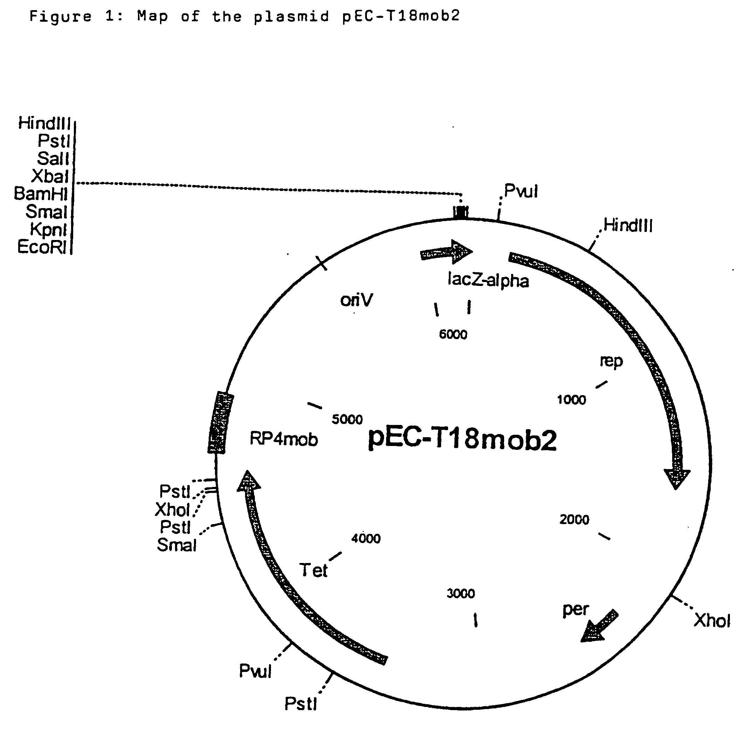

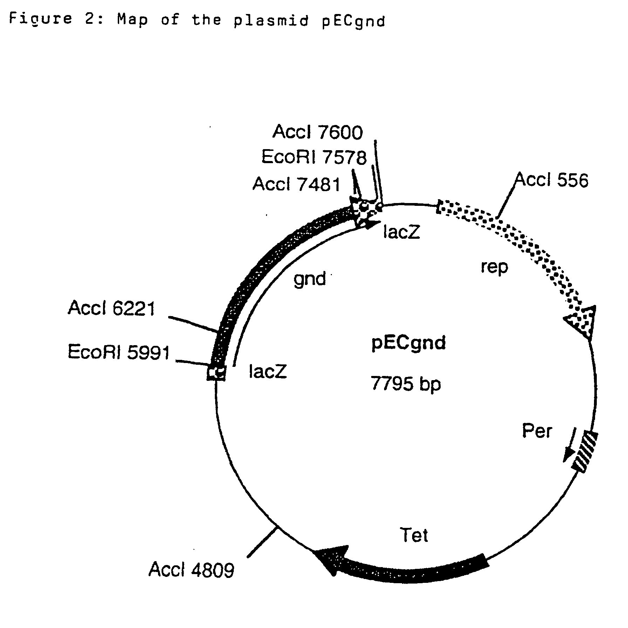

By way of example, 6-phosphogluconate dehydrogenase was over-expressed with the aid of a plasmid. The E. coli - C. glutamicum shuttle vector pEC-T18mob2 shown in Figure 1 was used for this. After incorporation of the gnd gene into the EcoRI cleavage site of pEC-T18mob2, the plasmid pECgnd shown in Figure 2 was formed.

Other plasmid vectors which are capable of replication in C. glutamicum, such as e.g. pEKExl (Eikmanns et al., Gene 102:93-98 (1991)) or pZ8-1 (EP-B- 0 375 889), can be used in the same way.

In addition, it may be advantageous for the production of L-amino acids to amplify one or more enzymes of the particular biosynthesis pathway, of glycolysis, of anaplerosis, of the pentose phosphate pathway or of amino By way of example, 6-phosphogluconate dehydrogenase was over-expressed with the aid of a plasmid. The E. coli - C. glutamicum shuttle vector pEC-T18mob2 shown in Figure 1 was used for this. After incorporation of the gnd gene into the EcoRI cleavage site of pEC-T18mob2, the plasmid pECgnd shown in Figure 2 was formed.

Other plasmid vectors which are capable of replication in C. glutamicum, such as e.g. pEKExl (Eikmanns et al., Gene 102:93-98 (1991)) or pZ8-1 (EP-B- 0 375 889), can be used in the same way.

In addition, it may be advantageous for the production of L-amino acids to amplify one or more enzymes of the particular biosynthesis pathway, of glycolysis, of anaplerosis, of the pentose phosphate pathway or of amino acid export, in addition to amplification of the gnd gene which codes for 6-phosphogluconate dehydrogenase and the attenuation of the pox B gene which codes for the pyruvate oxidase.

Thus, for example, in particular for the preparation of L-threonine, one or more genes chosen from the group consisting of
- the hom gene which codes for homoserine dehydrogenase (Peoples et al., Molecular Microbiology 2, 63-72 (1988)) or the hom^{dr} allele which codes for a "feed back resistant" homoserine dehydrogenase (Archer et al., Gene 107, 53-59 (1991),
- the gap gene which codes for glyceraldehyde 3-phosphate dehydrogenase (Eikmanns et al., Journal of Bacteriology 174: 6076-6086 (1992)),
- the pyc gene which codes for pyruvate carboxylase (Peters-Wendisch et al., Microbiology 144: 915-927 (1998)),
- the mqo gene which codes for malate:quinone oxidoreductase (Molenaar et al., European Journal of Biochemistry 254, 395-403 (1998)),
- the tkt gene which codes for transketolase (accession number AB023377 of the databank of European Molecular Biology Laboratories (EMBL, Heidelberg, Germany)),
- the zwf gene which codes for glucose 6-phosphate dehydrogenase (JP-A-09224661),
- the thrE gene which codes for threonine export (DE 199 41 478.5; DSM 12840),
can be amplified, in particular over-expressed, at the same time.

Thus, for example, in particular for the preparation of L-lysine, one or more genes chosen from the group consisting of
- the dapA gene which codes for dihydrodipicolinate synthase (EP-B 0 197 335),
- a lysC gene which codes for a feed back resistant aspartate kinase (Kalinowski et al. (1990), Molecular and General Genetics 224: 317-324),
- the gap gene which codes for glyceraldehyde 3-phosphate dehydrogenase (Eikmanns (1992), Journal of Bacteriology 174:6076-6086),
- the pyc gene which codes for pyruvate carboxylase (Eikmanns (1992), Journal of Bacteriology 174:6076-6086),
- the tkt gene which codes for transketolase (accession number AB023377 of the databank of European Molecular Biologies Laboratories (EMBL, Heidelberg, Germany)),
- the zwf gene which codes for glucose 6-phosphate dehydrogenase (JP-A-09224661),
- the lysE gene which codes for lysine export (DE-A-195 48 222),
can be amplified, in particular over-expressed, at the same time.

In addition to over-expression of 6-phosphogluconate dehydrogenase and attenuation of pyruvate oxidase, it may furthermore be advantageous for the production of L-amino acids to eliminate undesirable side reactions (Nakayama: "Breeding of Amino Acid Producing Micro-organisms", in: Overproduction of Microbial Products, Krumphanzl, Sikyta, Vanek. (eds.), Academic Press, London, UK, 1982).

The microorganisms prepared according to the invention can be cultured continuously or discontinuously in the batch process (batch culture) or in the fed batch (feed process) or repeated fed batch process (repetitive feed process) for the purpose of L-amino acid production. A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen [Bioreactors and Peripheral Equipment] (Vieweg Verlag, Braunschweig/Wiesbaden, 1994)).

The culture medium to be used must meet the requirements of the particular microorganisms in a suitable manner. Descriptions of culture media for various microorganisms are contained in the handbook "Manual of Methods for General Bacteriology" of the American Society for Bacteriology (Washington D.C., USA, 1981). Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture. Organic nitrogen-containing compounds, such as peptones, yeast extract, meat extract, malt extract, corn steep Basic compounds, such as sodium hydroxide, potassium hydroxide, ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of L-amino acid has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30, (1958), 1190), or it can take place by reversed phase HPLC as described by Lindroth et al. (Analytical Chemistry (1979) 51:. 1167-1174).

The following microorganism has been deposited at the Deutsche Sammlung für Mikroorganismen und Zellkulturen (DSMZ = German Collection of Microorganisms and Cell Cultures, Braunschweig, Germany) in accordance with the Budapest Treaty:
Escherichia coli K-12 DH5α/pEC-T18mob2 as DSM 13244

The following figures are attached:
- Figure 1: Map of the plasmid pEC-T18mob2
- Figure 2: Map of the plasmid pECgnd
- Figure 3: Map of the plasmid pBGNA
- Figure 4: Map of the plasmid pCR2.1poxBint

The base pair numbers stated are approx. values obtained in the context of reproducibility.

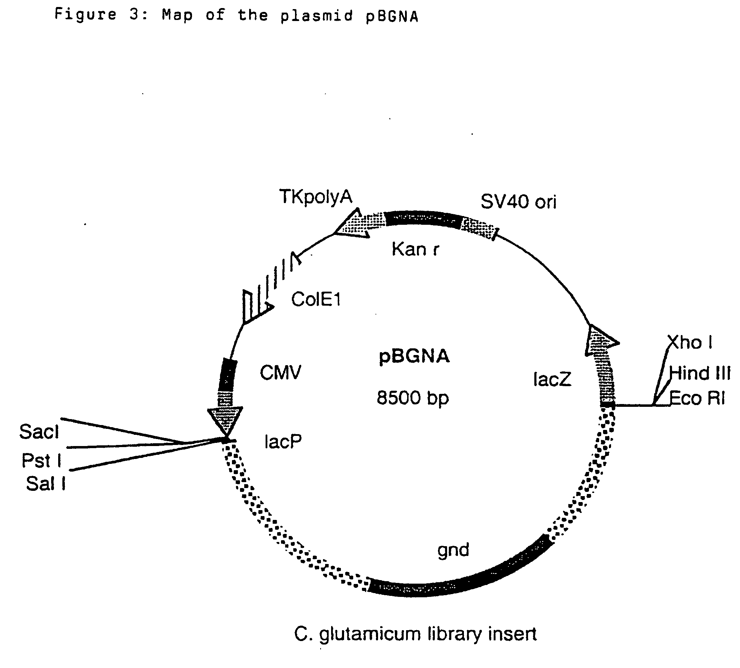

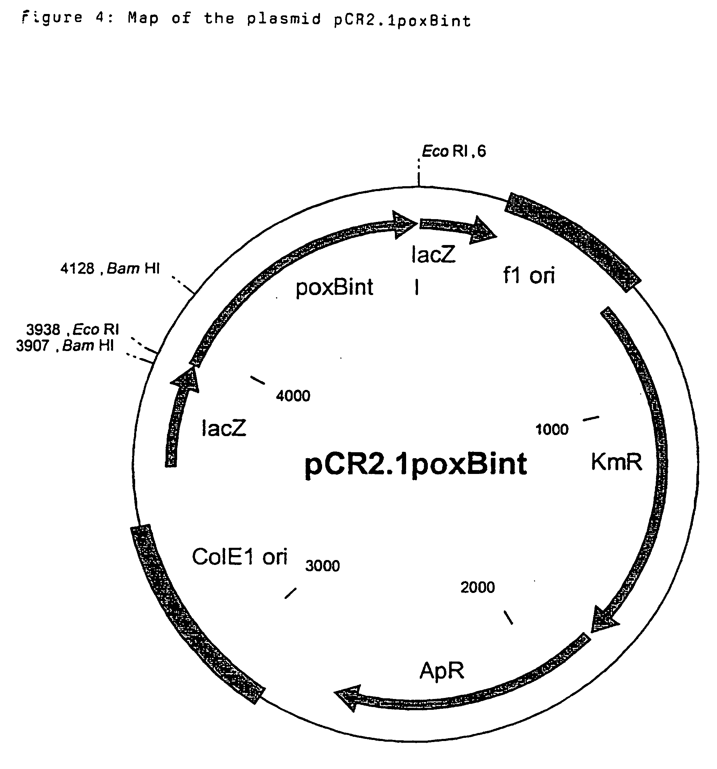

The abbreviations used have the following meaning:
Re Figure 1:
   - Tet:: Resistance gene for tetracycline
   - oriV:: Plasmid-coded replication origin of E. coli
   - RP4mob:: mob region for mobilizing the plasmid
   - rep:: Plasmid-coded replication origin from C. glutamicum plasmid pGA1
   - per:: Gene for controlling the number of copies from pGA1
   - lacZ-alpha:: lacZα gene fragment (N-terminus) of the β-Galactosidase gene
Re Figure 2:
   - Tet:: Resistance gene for tetracycline
   - rep:: Plasmid-coded replication origin from C. glutamicum plasmid pGA1
   - per:: Gene for controlling the number of copies from PGA1
   - lacZ: Cloning relict of the lacZα gene fragment from pEC-T18mob2
   - gnd:: 6-Phosphogluconate dehydrogenase gene
Re Figure 3:
   - LacP:: Promoter of the E. coli lactose operon
   - CMV:: Promoter of cytomegalovirus
   - ColE1:: Replication origin of the plasmid ColE1
   - TkpolyA:: Polyadenylation site
   - Kan r:: Kanamycin resistance gene
   - SV40ori:: Replication origin of Simian virus 40
   - gnd:: 6-Phosphogluconate dehydrogenase gene
Re Figure 4:
   - ColE1 ori:: Replication origin of the plasmid ColE1
   - lacZ:: Cloning relict of the lacZα gene fragment
   - fl ori:: Replication origin of phage f1
   - KmR:: Kanamycin resistance
   - ApR:: Ampicillin resistance
   - poxBint:: internal fragment of the poxB gene

Moreover, the following abbreviations have been used:
- AccI:: Cleavage site of the restriction enzyme AccI
- BamHI:: Cleavage site of the restriction enzyme BamHI
- EcoRI:: Cleavage site of the restriction enzyme EcoRI
- HindIII:: Cleavage site of the restriction enzyme HindIII
- KpnI:: Cleavage site of the restriction enzyme KpnI
- PstI:: Cleavage site of the restriction enzyme PstI
- PvuI:: Cleavage site of the restriction enzyme PvuI
- SalI:: Cleavage site of the restriction enzyme SalI
- SacI:: Cleavage site of the restriction enzyme SacI
- SmaI:: Cleavage site of the restriction enzyme SmaI
- SphI: Cleavage site of the restriction enzyme SphI
- XbaI:: Cleavage site of the restriction enzyme XbaI
- XhoI:: Cleavage site of the restriction enzyme XhoI

### Examples

The following examples will further illustrate this invention. The molecular biology techniques, e.g. plasmid DNA isolation, restriction enzyme treatment, ligations, standard transformations of Escherichia coli etc. used are, (unless stated otherwise), described by Sambrook et al., (Molecular Cloning. A Laboratory Manual (1989) Cold Spring Harbour Laboratories, USA).

### Example 1

### Construction of a gene library of Corynebacterium glutamicum strain AS019

A DNA library of Corynebacterium glutamicum strain ASO19 (Yoshihama et al., Journal of Bacteriology 162, 591-597 (1985)) was constructed using ë Zap Express™ system, (Short et al., (1988) Nucleic Acids Research 16: 7583-7600), as described by O'Donohue (O'Donohue, M. (1997). The Cloning and Molecular Analysis of Four Common Aromatic Amino Acid Biosynthetic Genes from Corynebacterium glutamicum. Ph.D. Thesis, National University of Ireland, Galway). ë Zap Express™ kit was purchased from Stratagene (Stratagene, 11011 North Torrey Pines Rd., La Jolla, California 92037) and used according to the manufacturers instructions. AS019-DNA was digested with restriction enzyme Sau3A and ligated to BamHI treated and dephosphorylated ë Zap Express™ arms.

### Example 2

### Cloning and sequencing of the gnd gene

### 1. Construction of a gnd probe

A radiolabelled oligonucleotide, internal to the gnd gene, was used to probe the AS019 ë Zap Express™ library described above. The oligonucleotide was produced using degenerate PCR primers internal to the gnd gene. The degenerate nucleotide primers designed for the PCR amplification of gnd DNA fragments were as follows: with R=A+G; Y=C+T; K=T+G.

The estimated size of the resulting PCR product was 252 bp approximately.

Optimal PCR conditions were determined to be as follows:
35 cycles
94°C for 1 minute
55°C for 1 minute
72°C for 30 seconds
2.5 - 3.5 mM MgCl₂
100 - 150 ng AS019 genomic DNA

Sequence analysis of the resulting PCR product confirmed the product to be an internal portion of a gnd gene. Sequence analysis was carried out using the universal forward and reverse primers, and T7 sequencing kit from Pharmacia Biotech, (St. Albans, Herts, UK). The sequence of the PCR product is shown in SEQ ID No. 1.

### 2. Cloning

Screening of the AS019 ë Zap Express™ library was carried out according to the ë Zap Express™ system protocol, (Stratagene, 11011 North Torrey Pines Rd., La Jolla, California 92037). Southern Blot analysis was then carried out on isolated clones. Southern transfer of DNA was as described in the Schleicher and Schuell protocols manual employing Nytran™ as membrane ("Nytran, Modified Nylon-66 Membrane Filters" (March 1987), Schleicher and Schuell, Dassel, Germany). Double stranded DNA fragments, generated using the same primers and optimal PCR conditions as described above, were radiolabelled with α-³²P-dCTP using the Multiprime™ DNA labelling kit from Amersham Life Science (Amersham Pharmacia Biotech UK Limited, Little Chalfont, Buckinghamshire, UK) according to the manufacturers instructions. Prehybridisation, hybridization and washing conditions were as described in the Schleicher and Schuell protocols manual. Autoradiography was carried out according to the procedure outlined in the handbook of Sambrook et al. using AgFa Curix RPIL film. Thus several gnd clones were identified. Plasmid DNA was isolated from one of the clones, designated pBGNA (Figure 3) and chosen for further analysis.

### 3. Sequencing

The Sanger Dideoxy chain termination method of Sanger et al. (Proceedings of the National Academy of Sciences USA 74, 5463-5467 (1977)) was used to sequence the cloned insert of pBGNA. The method was applied using the T7 sequencing kit and α-³⁵S-dCTP from Pharmacia Biotech (St. Albans, Herts, UK). Samples were electrophoresed for 3-8 hours on 6% polyacrylamide/urea gels in TBE buffer at a constant current of 50 mA, according to the Pharmacia cloning and sequencing instructions manual ("^{T7} Sequencing™ Kit",ref.XY-010-00-19, Pharmacia Biotech, 1994). Sequence analysis was carried out using internal primers designed from the sequence known of the internal gnd PCR product (SEQ ID NO 1) allowing the entire gnd gene sequence to be deduced.

The sequences of the internal primers were as follows:

Sequence obtained was then analyzed using the DNA Strider programme, (Marck (1988), Nucleic Acids Research 16: 1829-1836), version 1.0 on an Apple Macintosh computer. This program allowed for analyses such as restriction site usage, open reading frame analysis and codon usage determination. Searches between DNA sequence obtained and those in EMBL and Genbank databases were achieved using the BLAST programme (Altschul et al., (1997), Nucleic Acids Research 25: 3389-3402). DNA and protein sequences were aligned using the Clustal V and Clustal W programs (Higgins and Sharp, 1988 Gene 73: 237-244).

The sequence thus obtained is shown in SEQ ID NO 2. The analysis of the nucleotide sequence obtained revealed an open reading frame of 1377 base pairs which was designated as gnd gene. It codes for a protein of 459 amino acids shown in SEQ ID NO 3.

### Example 3

### Preparation of the shuttle vector pEC-T18mob2

The E. coli - C. glutamicum shuttle vector pEC-T18mob2 was constructed according to the prior art.

The vector contains the replication region rep of the plasmid pGA1 including the replication effector per (US-A-5,175,108; Nesvera et al., Journal of Bacteriology 179, 1525-1532 (1997)), the tetracycline resistance-imparting tetA(Z) gene of the plasmid pAG1 (US-A- 5,158,891; gene library entry at the National Center for Biotechnology Information (NCBI, Bethesda, MD, USA) with accession number AF121000), the replication region oriV of the plasmid pMB1 (Sutcliffe, Cold Spring Harbor Symposium on Quantitative Biology 43, 77-90 (1979)), the lacZ gene fragment including the lac promoter and a multiple cloning site (mcs) (Norrander et al. Gene 26, 101-106 (1983)) and the mob region of the plasmid RP4 (Simon et al., (1983) Bio/Technology 1:784-791).

The vector constructed was transformed in the E. coli strain DH5α (Hanahan, In: DNA cloning. A practical approach. Vol. I. IRL-Press, Oxford, Washington DC, USA, 1985). Selection for plasmid-carrying cells was made by plating out the transformation batch on LB agar (Sambrook et al., Molecular cloning: a laboratory manual. 2^{nd} Ed. Cold Spring Harbor Laboratory Press, Cold Spring Harbor, N.Y., USA, 1989), which had been supplemented with 5 mg/l tetracycline. Plasmid DNA was isolated from a transformant with the aid of the QIAprep Spin Miniprep Kit from Qiagen and checked by restriction with the restriction enzyme EcoRI and HindIII subsequent agarose gel electrophoresis (0.8%).

The plasmid was called pEC-T18mob2 and is shown in Figure 1. It is deposited in the form of the strain Escherichia coli K-12 strain DH5α/pEC-T18mob2 at the Deutsche Sammlung für Mikroorganismen und Zellkulturen (DSMZ = German Collection of Microorganisms and Cell Cultures, Braunschweig, Germany) as DSM 13244.

### Example 4

### Cloning of the gnd gene into the E. coli - C. glutamicum shuttle vector pEC-T18mob2

PCR was used to amplify DNA fragments containing the entire gnd gene of C. glutamicum and flanking upstream and downstream regions using pBGNA as template. PCR reactions were carried out using oligonucleotide primers designed from SEQ ID NO 2. The primers used were:

PCR parameters were as follows:
35 cycles
95°C for 6 minutes
94°C for 1 minute
50°C for 1 minute
72°C for 45 seconds
1 mM MgCl₂
approx. 150-200ng pBGNA-DNA as template.

The PCR product obtained was cloned into the commercially available pGEM-T vector purchased from Promega Corp. (pGEM-T Easy Vector System 1, cat. no. A1360, Promega UK, Southampton) using E. coli strain JM109 (Yanisch-Perron et al. Gene, 33: 103-119 (1985)) as a host. The entire gnd gene was subsequently isolated from the pGEM T-vector on an EcoRI fragment and cloned into the lacZ EcoRI site of the E. coli - C. glutamicum shuttle vector pEC-T18mob2 (Figure 1), and designated pECgnd (Figure 2). Restriction enzyme analysis with AccI (Boehringer Mannheim GmbH, Germany) revealed the correct orientation (i. e. downstream the lac-Promotor) of the gnd gene in the lacZα gene of pEC-T18mob2.

### Example 5

### Preparation of amino acid producers with amplified 6-phosphogluconate dehydrogenase

Plasmid pECgnd from Example 3 was electroporated by the electroporation method of Tauch et al. (FEMS Microbiological Letters, 123:343-347 (1994)) in the strains Corynebacterium glutamicum DSM 5399 and DSM 5714. The strain DSM 5399 is a threonine producer described in EP-B-0358940. The strain DSM 5714 is a lysine producer described in EP-B-0435132. Selection of transformants was carried out by plating out the electroporation batch on LB agar (Sambrook et al., Molecular cloning: a laboratory manual. 2^{nd} Ed. Cold Spring Harbor Laboratory Press, Cold Spring Harbor, N.Y., 1989), which had been supplemented with 25 mg/l kanamycin. The strains DSM5399/pECgnd and DSM5714/pECgnd were formed in this manner.

### Example 6

### Preparation of threonine

The C. glutamicum strain DSM5399/pECgnd obtained in Example 5 was cultured in a nutrient medium suitable for the production of threonine and the threonine content in the culture supernatant was determined.

For this, the strain was first incubated on an agar plate with the corresponding antibiotic (brain-heart agar with tetracycline (5 mg/l)) for 24 hours at 33°C. Starting from this agar plate culture, a preculture was seeded (10 ml medium in a 100 ml conical flask). Brain-heart broth (Merck, Darmstadt, Germany) was used as the medium for the preculture. Tetracycline (5 mg/l) was added to this medium. The preculture was incubated for 24 hours at 33°C at 240 rpm on a shaking machine. A main culture was seeded from this preculture such that the initial OD (660nm) of the main culture was 0.1. The medium MM-threonine was used for the main culture.

| Medium MM-threonine: | |
|---|---|
| CSL | 5 g/l |
| MOPS | 20 g/l |
| Glucose(autoclaved separately) | 50g/l |

| Salts: | |
|---|---|
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| CaCO₃ | 25 g/l |

The CSL (corn steep liquor), MOPS (morpholinopropanesulfonic acid) and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) was added. Culturing was carried out at 33°C and 80% atmospheric humidity.

After 48 hours, the OD was determined at a measurement wavelength of 660 nm with a Biomek 1000 (Beckmann Instruments GmbH, Munich). The concentration of threonine formed was determined with an amino acid analyzer from Eppendorf-BioTronik (Hamburg, Germany) by ion exchange chromatography and post-column derivatization with ninhydrin detection.

The result of the experiment is shown in Table 1.

**Table 1**

| Strain | OD(660) | L-Threonin g/l |
|---|---|---|
| DSM5399/pECgnd | 11.9 | 1.29 |
| DSM5399 | 11.8 | 0.33 |

### Example 7

### Preparation of lysine

The C. glutamicum strain DSM5714/pECgnd obtained in Example 5 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l)) for 24 hours at 33°C. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) was added to this medium. The preculture was incubated for 24 hours at 33°C at 240 rpm on a shaking machine. A main culture was seeded from this preculture such that the initial OD (660nm) of the main culture was 0.05. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 50g/l |
| (NH₄)₂SO₄ | |
| KH₂PO₄ | 25 g/l |
| MgSO₄ * 7 H₂O | 0.1 g/l |
| CaCl₂ * 2 H₂O | 1.0 g/l |
| FeSO₄ * 7 H₂O | 10 mg/l |
| MnSO₄ * H₂O | 10 mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) was added. Culturing was carried out at 33°C and 80% atmospheric humidity.

After 48 hours, the OD was determined at a measurement wavelength of 660 nm with a Biomek 1000 (Beckmann Instruments GmbH, München). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 2.

**Table 2**

| Strain | OD(660) | Lysine HCl g/l |
|---|---|---|
| DSM5715/pECgnd | 7.7 | 14.7 |
| DSM5715 | 7.1 | 13.7 |

### Example 8

### Preparation of a genomic cosmid gene library from Corynebacterium glutamicum ATCC 13032.

Chromosomal DNA from Corynebacterium glutamicum ATCC 13032 was isolated as described by Tauch et al., (1995, Plasmid 33:168-179), and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Molecular Biochemicals, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vektor Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase. The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no. 27-0870-04). The ligation mixture was then packed in phages with the aid of Gigapack II XL Packing Extracts (Stratagene, La Jolla, USA, Product Description Gigapack II XL Packing Extract, Code no. 200217). For infection of the E. coli strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology 1:190) + 100 µg/ml ampicillin. After incubation overnight at 37°C, recombinant individual clones were selected.

### Example 9

### Isolation and sequencing of the poxB gene

The cosmid DNA of an individual colony (Example 8) was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Molecular Biochemicals, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany). The DNA of the sequencing vector pZero-1, obtained from Invitrogen (Groningen, Holland, Product Description Zero Background Cloning Kit, Product No. K2500-01) was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology, 1:190) with 50 µg/ml zeocin. The plasmid preparation of the recombinant clones was carried out with Biorobot 9600 (Product No. 900200, Qiagen, Hilden, Germany). The sequencing was carried out by the dideoxy chain-stopping method of Sanger et al. (1977, Proceedings of the National Academies of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems(Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZerol derivatives were assembled to a continuous contig. The computer-assisted coding region analysis were prepared with the XNIP program (Staden, 1986, Nucleic Acids Research 14:217-231). Further analyses were carried out with the "BLAST search program" (Altschul et al., 1997, Nucleic Acids Research 25:3389-3402), against the non-redundant databank of the "National Center for Biotechnology Information" (NCBI, Bethesda, MD, USA).

The resulting nucleotide sequence is shown in SEQ ID No. 4. Analysis of the nucleotide sequence showed an open reading frame of 1737 base pairs, which was called the poxB gene. The poxB gene codes for a polypeptide of 579 amino acids (SEQ ID NO. 5).

### Example 10

### Preparation of an integration vector for integration mutagenesis of the poxB gene

From the strain ATCC 13032, chromosomal DNA was isolated by the method of Eikmanns et al. (Microbiology 140: 1817 - 1828 (1994)). On the basis of the sequence of the poxB gene known for C. glutamicum from Example 9, the following oligonucleotides were chosen for the polymerase chain reaction:

The primers shown were synthesized by MWG Biotech (Ebersberg, Germany) and the PCR reaction was carried out by the standard PCR method of Innis et al. (PCR protocols. A guide to methods and applications, 1990, Academic Press) with Pwo-Polymerase from Boehringer. With the aid of the polymerase chain reaction, a DNA fragment approx. 0.9 kb in size was isolated, this carrying an internal fragment of the poxB gene and being shown in SEQ ID No. 6.

The amplified DNA fragment was ligated with the TOPO TA Cloning Kit from Invitrogen Corporation (Carlsbad, CA, USA; Catalogue Number K4500-01) in the vector pCR2.1-TOPO (Mead at al. (1991) Bio/Technology 9:657-663). The E. coli Stamm DH5α was then electroporated with the ligation batch (Hanahan, In: DNA cloning. A practical approach. Vol.I. IRL-Press, Oxford, Washington DC, USA, 1985). Selection for plasmid-carrying cells was made by plating out the transformation batch on LB agar (Sambrook et al., Molecular cloning: a laboratory manual. 2^{nd} Ed. Cold Spring Harbor Laboratory Press, Cold Spring Harbor, N.Y., 1989), which had been supplemented with 25 mg/l kanamycin. Plasmid DNA was isolated from a transformant with the aid of the QIAprep Spin Miniprep Kit from Qiagen and checked by restriction with the restriction enzyme EcoRI and subsequent agarose gel electrophoresis (0.8%). The plasmid was called pCR2.1poxBint (Figure 4).

Plasmid pCR2.1poxBint has been deposited in the form of the strain Escherichia coli DH5α/pCR2.1poxBint as DSM 13114 at the Deutsche Sammlung für Mikroorganismen und Zellkulturen (DSMZ = German Collection of Microorganisms and Cell Cultures, Braunschweig, Germany) in accordance with the Budapest Treaty.

### Example 11

### Integration mutagenesis of the poxB gene in the lysine producer DSM 5715

The vector pCR2.1poxBint mentioned in Example 10 was electroporated by the electroporation method of Tauch et al.(FEMS Microbiological Letters, 123:343-347 (1994)) in Corynebacterium glutamicum DSM 5715. Strain DSM 5715 is an AEC-resistant lysine producer. The vector pCR2.1poxBint cannot replicate independently in DSM5715 and is retained in the cell only if it has integrated into the chromosome of DSM 5715. Selection of clones with pCR2.1poxBint integrated into the chromosome was carried out by plating out the electroporation batch on LB agar (Sambrook et al., Molecular cloning: a laboratory manual. 2^{nd} Ed. Cold Spring Harbor Laboratory Press, Cold Spring Harbor, N.Y.), which had been supplemented with 15 mg/l kanamycin. For detection of the integration, the poxBint fragment was labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant was isolated by the method of Eikmanns et al. (Microbiology 140: 1817 - 1828 (1994)) and in each case cleaved with the restriction enzymes SalI, SacI and HindIII. The fragments formed were separated by agarose gel electrophoresis and hybridized at 68°C with the Dig hybrization kit from Boehringer. The plasmid pCR2.1poxBint mentioned in Example 9 had been inserted into the chromosome of DSM5715 within the chromosomal poXB gene. The strain was called DSM5715::pCR2.1poxBint.

### Example 12

### Effect of over-expression of the gnd gene with simultaneous elimination of the poxB gene on the preparation of lysine

### 12.1 Preparation of the strain

DSM5715::pCR2.1poxBint/pECgnd

The strain DSM5715::pCR2.1poxBint was transformed with the plasmid pECgnd using the electroporation method described by Liebl et al., (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 5 mg/l tetracycline and 25 mg/l kanamycin. Incubation was carried out for 2 days at 33°C.

Plasmid DNA was isolated in each case from a transformant by conventional methods (Peters-Wendisch et al., 1998, Microbiology 144, 915 -927), cleaved with the restriction endonuclease AccI, and the plasmid was checked by subsequent agarose gel electrophoresis. The strain obtained in this way was called DSM5715:pCR2.1poxBint/pECgnd.

### 12.2 Preparation of L-lysine

The C. glutamicum strain DSM5715::pCR2.1poxBint/pECgnd obtained in Example 12.1 was cultured in a nutrient medium suitable for the production of lysine and the lysine content in the culture supernatant was determined.

For this, the strain was first incubated on an agar plate with the corresponding antibiotic (brain-heart agar with tetracycline (5 mg/l) and kanamycin (25 mg/l)) for 24 hours at 33°C. The cultures of the comparison strains were supplemented according to their resistance to antibiotics. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Tetracycline (5 mg/l) and kanamycin (25 mg/l) were added to this. The preculture was incubated for 16 hours at 33°C at 240 rpm on a shaking machine. A main culture was seeded from this preculture such that the initial OD (660nm) of the main culture was 0.1. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 58 g/l |

| | |
|---|---|
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Tetracycline (5 mg/l) and kanamycin (25 mg/l) were added. Culturing was carried out at 33°C and 80% atmospheric humidity.

After 72 hours, the OD was determined at a measurement wavelength of 660 nm with a Biomek 1000 (Beckmann Instruments GmbH, München). The amount of lysine formed was determined with an amino acid analyzer from Eppendorf-BioTronik (Hamburg, Germany) by ion exchange chromatography and post-column derivatization with ninhydrin detection.

The result of the experiment is shown in Table 3.

**Table 3**

| Strain | OD | L-Lysine HCl g/l |
|---|---|---|
| DSM5715 | 10.8 | 16.0 |
| DSM5715/pECgnd | 7.6 | 16.5 |
| DSM5715::pCR2.1poxBint | 7.1 | 16.7 |
| DSM5715::pCR2.1poxBint/ pECgnd | 7.2 | 17.1 |

## Claims

1. A process for the preparation of L-amino acids by fermentation of coryneform bacteria which comprises carrying out the following steps:
a) fermentation of the desired L-amino acid-producing bacteria in which at least the gnd gene is amplified and at least the poxB gene is attenuated,
b) concentration of the L-amino acid in the medium or in the cells of the bacteria and
c) isolation of the L-amino acid produced.

2. The process as claimed in claim 1, wherein bacteria in which further genes of the biosynthesis pathway of the desired L-amino acid are additionally amplified, in particular over-expressed, are employed.

3. The process as claimed in claim 1, wherein coryneform bacteria which prepare L-threonine, L-lysine, L-isoleucine or L-tryptophan are used.

4. The process as claimed in claim 3, wherein coryneform bacteria which prepare L-lysine are used.

5. The process as claimed in claim 3, wherein coryneform bacteria which prepare L-threonine are used.

6. A process for the fermentative preparation of L-lysine as claimed in claim 2, wherein in the coryneform microorganisms which in particular already produce L-lysine, one or more genes chosen from the group consisting of
6.1 the dapA gene which codes for dihydrodipicolinate synthase,
6.2 the lysC gene which codes for a feed back resistant aspartate kinase,
6.3 the gap gene which codes for glyceraldehyde 3-phosphate dehydrogenase,
6.4 the pyc gene which codes for pyruvate carboxylase,
6.5 the tkt gene which codes for transketolase,
6.6 the zwf gene which codes for glucose 6-phosphate dehydrogenase, and
6.7 the lysE gene which codes for lysine export,
is or are amplified, in particular over-expressed, at the same time.

7. A process for the fermentative preparation of L-threonine as claimed in claim 2, wherein in the coryneform microorganisms which in particular already produce L-threonine, one or more genes chosen from the group consisting of
7.1 the hom gene which codes for homoserine dehydrogenase or the hom^{dr} allele which codes for a "feed back resistant" homoserine dehydrogenase,
7.2 the gap gene which codes for glyceraldehyde 3-phosphate dehydrogenase,
7.3 the pyc gene which codes for pyruvate carboxylase,
7.4 the mqo gene which codes for malate:quinone oxidoreductase,
7.5 the tkt gene which codes for transketolase,
7.6 the zwf gene which codes for glucose 6-phosphate dehydrogenase, and
7.7 the thrE gene which codes for threonine export,
is or are amplified, in particular over-expressed, at the same time.

8. The process as claimed in claims 2 to 7, wherein to achieve the amplification, the number of copies of the genes or nucleotide sequences is increased by transformation of the microorganisms with plasmid vectors which carry these genes or nucleotide sequences.

## Patentansprüche

1. Verfahren zur Herstellung von L-Aminosäuren durch Fermentation von Coryneform-Bakterium, umfassend die Ausführung der folgenden Schritte:
a) Fermentieren der gewünschten L-Aminosäureproduzierenden Bakterien, in denen wenigstens das gnd-Gen amplifiziert und wenigstens das poxB-Gen attenuiert wird,
b) Konzentrieren der L-Aminosäure im Medium oder in den Zellen der Bakterien und
c) Isolieren der produzierten L-Aminosäure.

2. Verfahren nach Anspruch 1, bei dem Bakterien, in denen weitere Gene des Biosyntheseweges der gewünschten L-Aminosäure zusätzlich amplifiziert, insbesondere überexprimiert werden, verwendet werden.

3. Verfahren nach Anspruch 1, bei dem Coryneform-Bakterien, die L-Threonin, L-Lysin, L-Isoleucin oder L-Tryptophan herstellen, verwendet werden.

4. Verfahren nach Anspruch 3, bei dem Coryneform-Bakterien, die L-Lysin herstellen, verwendet werden.

5. Verfahren nach Anspruch 3, bei dem Coryneform-Bakterien, die L-Threonin herstellen, verwendet werden.

6. Verfahren zur fermentativen Herstellung von L-Lysin nach Anspruch 2, wobei in den Coryneform-Mikroorganismen, die insbesondere bereits L-Lysin produzieren, ein oder mehrere Gene ausgewählt aus der Gruppe bestehend aus:
6.1 dem dapA-Gen, das Dihydrodipicolinatsynthase codiert,
6.2 dem lysC-Gen, das feedbackresistente Aspartatkinase codiert,
6.3 dem gap-Gen, das Glyceraldehyd-3-phosphatdehydrogenase codiert,
6.4 dem pyc-Gen, das Pyruvatcarboxylase codiert,
6.5 dem tkt-Gen, das Transketolase codiert,
6.6 dem zwf-Gen, das Glucose-6-phosphatdehydrogenase codiert, und
6.7 dem lysE-Gen, das Lysinexport codiert,
gleichzeitig amplifiziert, insbesondere überexprimiert, wird oder werden.

7. Verfahren zur fermentativen Herstellung von L-Threonin nach Anspruch 2, wobei in den Coryneform-Mikroorganismen, die insbesondere bereits L-Threonin produzieren, ein oder mehrere Gene ausgewählt aus der Gruppe bestehend aus:
7.1 dem hom-Gen, das Homoserindehydrogenase codiert, oder dem hom^{dr}-Allel, das eine "feedbackresistente" Homoserindehydrogenase codiert,
7.2 dem gap-Gen, das Glyeraldehyd-3-phosphatdehydrogenase codiert,
7.3 dem pyc-Gen, das Pyruvatcarboxylase codiert,
7.4 dem mqo-Gen, das Malat:Chinon-Oxidoreductase codiert,
7.5 dem tkt-Gen, das Transketolase codiert,
7.6 dem zwf-Gen, das Glucose-6-phospatdehydrogenase codiert, und
7.7 dem thrE-Gen, das Threoninexport codiert,
gleichzeitig amplifiziert, insbesondere überexprimiert, wird oder werden.

8. Verfahren nach einem der Ansprüche 2 bis 7, wobei zum Erzielen der Amplifizierung die Anzahl von Kopien der Gene oder Nucleotidsequenzen durch Transformation der Mikroorganismen mit Plasmidvektoren erhöht wird, die diese Gene oder Nucleotidsequenzen tragen.

## Revendications

1. Procédé pour la préparation d'acides aminés L par une fermentation de bactéries de type coryneforme qui comprend la réalisation des étapes suivantes :
a) fermentation des bactéries produisant l'acide aminé L souhaité dans lesquelles au moins le gène gnd est amplifié et au moins le gène poxB est atténué,
b) concentration de l'acide aminé L dans le milieu ou dans les cellules des bactéries et
c) isolation de l'acide aminé-L produit.

2. Procédé selon la revendication 1, dans lequel on utilise des bactéries dans lesquelles d'autres gènes de la voie de biosynthèse de l'acide aminé-L souhaité sont additionnellement amplifiés, en particulier surexprimés.

3. Procédé selon la revendication 1, dans lequel on utilise des bactéries de type coryneforme qui préparent de la L-thréonine, de la L-lysine, de la L-isoleucine ou du L-tryptophane.

4. Procédé selon la revendication 3, dans lequel on utilise des bactéries de type coryneforme qui préparent de la L-lysine.

5. Procédé selon la revendication 3, dans lequel on utilise des bactéries de type coryneforme qui préparent de la L-thréonine.

6. Procédé pour la préparation par fermentation de la L-lysine selon la revendication 2, dans lequel, dans les microorganismes de type coryneforme qui en particulier produisent déjà de la L-lysine, un ou plusieurs gènes choisis dans le groupe constitué :
6.1 du gène dapA qui code pour une dihydrodipicolinate synthétase,
6.2 du gène lysC qui code pour une aspartate kinase résistante à l'inhibition rétroactive,
6.3 du gène gap qui code pour une glycéraldéhyde 3-phosphate déshydrogénase,
6.4 du gène pyc qui code pour une pyruvate carboxylase,
6.5 du gène tkt qui code pour une transcétolase
6.6 du gène zwf qui code pour une glucose 6-phosphate déshydrogénase, et
6.7 du gène lyse qui code pour une exportation de la lysine,
est ou sont simultanément ampliflé(s), en particulier surexprimé(s).

7. Procédé pour la préparation par fermentation de la L-thréonine selon la revendication 2, dans lequel, dans les microorganismes de type coryneforme qui en particulier produisent déjà de la L-thréonine, un ou plusieurs gènes choisis dans le groupe constitué :
7.1 du gène hom qui code pour une homosérine déshydrogénase ou de l'allèle hom^{dr} qui code pour une homosérine déshydrogénase «résistante à l'inhibition rétroactive»,
7.2 du gène gap qui code pour la glycéraldéhyde 3-phosphate déshydrogénase,
7.3 du gène pyc qui code pour une pyruvate carboxylase,
7.4 du gène mqo qui code pour une malate/quinone oxydoréductase,
7.5 du gène tkt qui code pour une transcétolase,
7.6 du gène zwf qui code pour une glucose 6-phosphate déshydrogénase, et
7,7 du gène thrE qui code pour une exportation de la thréonine,
est ou sont simultanément amplifié(s), en particulier surexprimé(s).

8. Procédé selon les revendications 2 à 7, dans lequel, pour réaliser l'amplification, on augmente le nombre de copies des gènes ou des séquences nucléotidiques par une transformation des microorganismes avec des vecteurs plasmidiques qui portent ces gènes ou séquences nucléotidiques.


### SEQUENCE PROTOCOL

<110> National University of Ireland, Galway Degussa-Hüls AG
<120> Process for the fermentative preparation of L-amino acids using coryneform bacteria.
<130> 990229 BT
<140>
   <141>
<160> 6
<170> PatentIn Ver. 2.1
<210> 1
   <211> 252
   <212> DNA
   <213> Corynebacterium glutamicum
<400> 1
   atggtccaca acggcatcga gtacgccgac atgcaggtca tcggcgaggc ataccacctt 60
   ctgccctacg cagcaggcat gcagccagct gaaatcgctg aggttttcaa ggaatggaac 120
   gcaggcgacc tggattccta cctcatcgaa atcaccgcag aggttctctc ccaggtggat 180
   gctgaaaccg gcaagccact aatcgacgtc atcgttgacg ctgcaggtca gaagggcacc 240
   ggcaagtgga ct                                                     252
<210> 2
   <211> 2335
   <212> DNA
   <213> Corynebacterium glutamicum
<220>
   <221> CDS
   <222> (474)..(1850)
   <223> gnd
<400> 2
   ttgttcggcc acgatgacac cggagctcac agcagaaatg aagtcggtgt tgttgttgat 60
   gccgacgacc atttttccag gggcggaaat catgctggcg actgatccag tggattcggc 120
   gatggcggcg tagacaccac cgttgaccaa gcccaccact tgcaggtgct tggatgccac 180
   gtgaagttcg ctgaccaccc ggccgggctc gatggtggtg tagcgcagcc ccagattgcg 240
   gtcgaggcca taattggcgt tgttgagtgc ttcaagttcg tctgtggtta aagctctggt 300
   ggcggcaagt tctgcaagcg aaagcagatc ttggggttga tcatcgcggg aagtcataat 360
   taattactct agtcggccta aaatggttgg attttcacct cctgtgacct ggtaaaatcg 420
   ccactacccc caaatggtca caccttttag gccgattttg ctgacaccgg gct atg    476
                                                              Met
                                                               1
   ccg tca agt acg atc aat aac atg act aat gga gat aat ctc gca cag   524
   Pro Ser Ser Thr Ile Asn Asn Met Thr Asn Gly Asp Asn Leu Ala Gln
               5                   10                  15
   atc ggc gtt gta ggc cta gca gta atg ggc tca aac ctc gcc cgc aac    572
   Ile Gly Val Val Gly Leu Ala Val Met Gly Ser Asn Leu Ala Arg Asn
        20                  25                  30
   ttc gcc cgc aac ggc aac act gtc gct gtc tac aac cgc agc act gac    620
   Phe Ala Arg Asn Gly Asn Thr Val Ala Val Tyr Asn Arg Ser Thr Asp
        35                  40                  45
   aaa acc gac aag ctc atc gcc gat cac ggc tcc gaa ggc aac ttc atc    668
   Lys Thr Asp Lys Leu Ile Ala Asp His Gly Ser Glu Gly Asn Phe Ile
    50                  55                  60                  65
   cct tct gca acc gtc gaa gag ttc gta gca tcc ctg gaa aag cca cgc    716
   Pro Ser Ala Thr Val Glu Glu Phe Val Ala Ser Leu Glu Lys Pro Arg
                    70                  75                  80
   cgc gcc atc atc atg gtt cag gct ggt aac gcc acc gac gca gtc atc    764
   Arg Ala Ile Ile Met Val Gln Ala Gly Asn Ala Thr Asp Ala Val Ile
                85                  90                  95
   aac cag ctg gca gat gcc atg gac gaa ggc gac atc atc atc gac ggc    812
   Asn Gln Leu Ala Asp Ala Met Asp Glu Gly Asp Ile Ile Ile Asp Gly
           100                 105                 110
   ggc aac gcc ctc tac acc gac acc att cgt cgc gag aag gaa atc tcc    860
   Gly Asn Ala Leu Tyr Thr Asp Thr Ile Arg Arg Glu Lys Glu Ile Ser
       115                 120                 125
   gca cgc ggt ctc cac ttc gtc ggt gct ggt atc tcc ggc ggc gaa gaa    908
   Ala Arg Gly Leu His Phe Val Gly Ala Gly Ile Ser Gly Gly Glu Glu
   130                 135                 140                 145
   ggc gca ctc aac ggc cca tcc atc atg cct ggt ggc cca gca aag tcc    956
   Gly Ala Leu Asn Gly Pro Ser Ile Met Pro Gly Gly Pro Ala Lys Ser
                   150                 155                 160
   tac gag tcc ctc gga cca ctg ctt gag tcc atc gct gcc aac gtt gac   1004
   Tyr Glu Ser Leu Gly Pro Leu Leu Glu Ser Ile Ala Ala Asn Val Asp
               165                 170                 175
   ggc acc cca tgt gtc acc cac atc ggc cca gac ggc gcc ggc cac ttc   1052
   Gly Thr Pro Cys Val Thr His Ile Gly Pro Asp Gly Ala Gly His Phe
           180                 185                 190
   gtc aag atg gtc cac aac ggc atc gag tac gcc gac atg cag gtc atc   1100
   Val Lys Met Val His Asn Gly Ile Glu Tyr Ala Asp Met Gln Val Ile
       195                 200                 205
   ggc gag gca tac cac ctt ctg ccc tac gca gca ggc atg cag cca gct   1148
   Gly Glu Ala Tyr His Leu Leu Pro Tyr Ala Ala Gly Met Gln Pro Ala
   210                 215                 220                 225
   gaa atc gct gag gtt ttc aag gaa tgg aac gca ggc gac ctg gat tcc   1196
   Glu Ile Ala Glu Val Phe Lys Glu Trp Asn Ala Gly Asp Leu Asp Ser
                   230                 235                 240
   tac ctc atc gaa atc acc gca gag gtt ctc tcc cag gtg gat gct gaa   1244
   Tyr Leu Ile Glu Ile Thr Ala Glu Val Leu Ser Gln Val Asp Ala Glu
               245                 250                 255
   acc ggc aag cca cta atc gac gtc atc gtt gac gct gca ggt cag aag   1292
   Thr Gly Lys Pro Leu Ile Asp Val Ile Val Asp Ala Ala Gly Gln Lys
           260                 265                 270
   ggc acc ggc aag tgg act gtc aag gct gct ctt gat ctg ggt att gct     1340
   Gly Thr Gly Lys Trp Thr Val Lys Ala Ala Leu Asp Leu Gly Ile Ala
       275                 280                 285
   acc acc ggc atc ggc gaa cgt gtt ttc gca cgt gca ctc tcc ggc gca     1388
   Thr Thr Gly Ile Gly Glu Arg Val Phe Ala Arg Ala Leu Ser Gly Ala
   290                 295                 300                 305
   acc agc cag cgc gct gca gca cag ggc aac cta cct gca ggt gtc ctc     1436
   Thr Ser Gln Arg Ala Ala Ala Gln Gly Asn Leu Pro Ala Gly Val Leu
                   310                 315                 320
   acc gat ctg gaa gca ctt ggc gtg gac aag gca cag ttc gtc gaa gga     1484
   Thr Asp Leu Glu Ala Leu Gly Val Asp Lys Ala Gln Phe Val Glu Gly
               325                 330                 335
   ctt cgc cgt gca ctg tac gca tcc aag ctt gtt gct tac gca cag ggc     1532
   Leu Arg Arg Ala Leu Tyr Ala Ser Lys Leu Val Ala Tyr Ala Gln Gly
           340                 345                 350
   ttc gac gag atc aag gct ggc tcc gac gag aac aac tgg gac gtt gac     1580
   Phe Asp Glu Ile Lys Ala Gly Ser Asp Glu Asn Asn Trp Asp Val Asp
       355                 360                 365
   cct cgc gac ctc gct acc atc tgg cgc ggc ggc tgc atc att cgc gct     1628
   Pro Arg Asp Leu Ala Thr Ile Trp Arg Gly Gly Cys Ile Ile Arg Ala
   370                 375                 380                 385
   aag ttc ctc aac cgc atc gtc gaa gca tac gat gca aac gct gaa ctt     1676
   Lys Phe Leu Asn Arg Ile Val Glu Ala Tyr Asp Ala Asn Ala Glu Leu
                   390                 395                 400
   gag tcc ctg ctg ctc gat cct tac ttc aag agc gag ctc ggc gac ctc     1724
   Glu Ser Leu Leu Leu Asp Pro Tyr Phe Lys Ser Glu Leu Gly Asp Leu
               405                 410                 415
   atc gat tca tgg cgt cgc gtg att gtc acc gcc acc cag ctt ggc ctg     1772
   Ile Asp Ser Trp Arg Arg Val Ile Val Thr Ala Thr Gln Leu Gly Leu
           420                 425                 430
   cca atc cca gtg ttc gct tcc tcc ctg tcc tac tac gac agc ctg cgt     1820
   Pro Ile Pro Val Phe Ala Ser Ser Leu Ser Tyr Tyr Asp Ser Leu Arg
       435                 440                 445
   gca gag cgt ctg cca gca gcc ctg atc cac tagtgtcgac ctgcaggcgc       1870
   Ala Glu Arg Leu Pro Ala Ala Leu Ile His
   450                 455
   gcgagctcca gcttttgttc cctttagtga gggttaattt cgagcttggc gtaatcaagg 1930
   tcatagctgt ttcctgtgtg aaattgttat ccgctcacaa ttccacacaa tatacgagcc 1990
   ggaagtataa agtgtaaagc ctggggtgcc taatgagtga gctaactcac agtaattgcg 2050
   gctagcggat ctgacggttc actaaaccag ctctgcttat atagacctcc caccgtacac 2110
   gcctaccgcc catttgcgtc aatggggcgg agttgttacg acattttgga aagtcccgtt 2170
   gattttggtg ccaaaacaaa ctcccattga cgtcaatggg gtggagactt ggaaatcccc 2230
   gtgagtcaaa ccgctatcca cgcccattga tgtactgcca aaaccgcatc accatggtaa 2290
   tagcgatgac taatacgtag atgtactgcc aagtaggaaa gtccc 2335
<210> 3
   <211> 459
   <212> PRT
   <213> Corynebacterium glutamicum
<400> 3
   Met Pro Ser Ser Thr Ile Asn Asn Met Thr Asn Gly Asp Asn Leu Ala
   1 5 10 15
   Gln Ile Gly Val Val Gly Leu Ala Val Met Gly Ser Asn Leu Ala Arg
   20 25 30
   Asn Phe Ala Arg Asn Gly Asn Thr Val Ala Val Tyr Asn Arg Ser Thr
   35 40 45
   Asp Lys Thr Asp Lys Leu Ile Ala Asp His Gly Ser Glu Gly Asn Phe
   50 55 60
   Ile Pro Ser Ala Thr Val Glu Glu Phe Val Ala Ser Leu Glu Lys Pro
   65 70 75 80
   Arg Arg Ala Ile Ile Met Val Gln Ala Gly Asn Ala Thr Asp Ala Val
   85 90 95
   Ile Asn Gln Leu Ala Asp Ala Met Asp Glu Gly Asp Ile Ile Ile Asp
   100 105 110
   Gly Gly Asn Ala Leu Tyr Thr Asp Thr Ile Arg Arg Glu Lys Glu Ile
   115 120 125
   Ser Ala Arg Gly Leu His Phe Val Gly Ala Gly Ile Ser Gly Gly Glu
   130 135 140
   Glu Gly Ala Leu Asn Gly Pro Ser Ile Met Pro Gly Gly Pro Ala Lys
   145 150 155 160
   Ser Tyr Glu Ser Leu Gly Pro Leu Leu Glu Ser Ile Ala Ala Asn Val
   165 170 175
   Asp Gly Thr Pro Cys Val Thr His Ile Gly Pro Asp Gly Ala Gly His
   180 185 190
   Phe Val Lys Met Val His Asn Gly Ile Glu Tyr Ala Asp Met Gln Val
   195 200 205
   Ile Gly Glu Ala Tyr His Leu Leu Pro Tyr Ala Ala Gly Met Gln Pro
   210 215 220
   Ala Glu Ile Ala Glu Val Phe Lys Glu Trp Asn Ala Gly Asp Leu Asp
   225 230 235 240
   Ser Tyr Leu Ile Glu Ile Thr Ala Glu Val Leu Ser Gln Val Asp Ala
   245 250 255
   Glu Thr Gly Lys Pro Leu Ile Asp Val Ile Val Asp Ala Ala Gly Gln
   260 265 270
   Lys Gly Thr Gly Lys Trp Thr Val Lys Ala Ala Leu Asp Leu Gly Ile
   275 280 285
   Ala Thr Thr Gly Ile Gly Glu Arg Val Phe Ala Arg Ala Leu Ser Gly
   290 295 300
   Ala Thr Ser Gln Arg Ala Ala Ala Gln Gly Asn Leu Pro Ala Gly Val
   305 310 315 320
   Leu Thr Asp Leu Glu Ala Leu Gly Val Asp Lys Ala Gln Phe Val Glu
   325 330 335
   Gly Leu Arg Arg Ala Leu Tyr Ala Ser Lys Leu Val Ala Tyr Ala Gln
   340 345 350
   Gly Phe Asp Glu Ile Lys Ala Gly Ser Asp Glu Asn Asn Trp Asp Val
   355 360 365
   Asp Pro Arg Asp Leu Ala Thr Ile Trp Arg Gly Gly Cys Ile Ile Arg
   370 375 380
   Ala Lys Phe Leu Asn Arg Ile Val Glu Ala Tyr Asp Ala Asn Ala Glu
   385 390 395 400
   Leu Glu Ser Leu Leu Leu Asp Pro Tyr Phe Lys Ser Glu Leu Gly Asp
   405 410 415
   Leu Ile Asp Ser Trp Arg Arg Val Ile Val Thr Ala Thr Gln Leu Gly
   420 425 430
   Leu Pro Ile Pro Val Phe Ala Ser Ser Leu Ser Tyr Tyr Asp Ser Leu
   435 440 445
   Arg Ala Glu Arg Leu Pro Ala Ala Leu Ile His
   450 455
<210> 4
   <211> 2160
   <212> DNA
   <213> Corynebacterium glutamicum
<220>
   <221> CDS
   <222> (327)..(2063)
   <223> poxB
<400> 4
   ttagaggcga ttctgtgagg tcactttttg tggggtcggg gtctaaattt ggccagtttt 60
   cgaggcgacc agacaggcgt gcccacgatg tttaaatagg cgatcggtgg gcatctgtgt 120
   ttggtttcga cgggctgaaa ccaaaccaga ctgcccagca acgacggaaa tcccaaaagt 180
   gggcatccct gtttggtacc gagtacccac ccgggcctga aactccctgg caggcgggcg 240
   aagcgtggca acaactggaa tttaagagca caattgaagt cgcaccaagt taggcaacac 300
   aatagccata acgttgagga gttcag atg gca cac agc tac gca gaa caa tta  353
                                Met Ala His Ser Tyr Ala Glu Gln Leu
                                 1               5
   att gac act ttg gaa gct caa ggt gtg aag cga att tat ggt ttg gtg   401
   Ile Asp Thr Leu Glu Ala Gln Gly Val Lys Arg Ile Tyr Gly Leu Val
    10                  15                  20                  25
   ggt gac agc ctt aat ccg atc gtg gat gct gtc cgc caa tca gat att      449
   Gly Asp Ser Leu Asn Pro Ile Val Asp Ala Val Arg Gln Ser Asp Ile
                   30                  35                  40
   gag tgg gtg cac gtt cga aat gag gaa gcg gcg gcg ttt gca gcc ggt      497
   Glu Trp Val His Val Arg Asn Glu Glu Ala Ala Ala Phe Ala Ala Gly
               45                  50                  55
   gcg gaa tcg ttg atc act ggg gag ctg gca gta tgt gct gct tct tgt      545
   Ala Glu Ser Leu Ile Thr Gly Glu Leu Ala Val Cys Ala Ala Ser Cys
           60                  65                  70
   ggt cct gga aac aca cac ctg att cag ggt ctt tat gat tcg cat cga      593
   Gly Pro Gly Asn Thr His Leu Ile Gln Gly Leu Tyr Asp Ser His Arg
       75                  80                  85
   aat ggt gcg aag gtg ttg gcc atc gct agc cat att ccg agt gcc cag      641
   Asn Gly Ala Lys Val Leu Ala Ile Ala Ser His Ile Pro Ser Ala Gln
   90                  95                  100                 105
   att ggt tcg acg ttc ttc cag gaa acg cat ccg gag att ttg ttt aag      689
   Ile Gly Ser Thr Phe Phe Gln Glu Thr His Pro Glu Ile Leu Phe Lys
                   110                 115                 120
   gaa tgc tct ggt tac tgc gag atg gtg aat ggt ggt gag cag ggt gaa      737
   Glu Cys Ser Gly Tyr Cys Glu Met Val Asn Gly Gly Glu Gln Gly Glu
               125                 130                 135
   cgc att ttg cat cac gcg att cag tcc acc atg gcg ggt aaa ggt gtg      785
   Arg Ile Leu His His Ala Ile Gln Ser Thr Met Ala Gly Lys Gly Val
           140                 145                 150
   tcg gtg gta gtg att cct ggt gat atc gct aag gaa gac gca ggt gac      833
   Ser Val Val Val Ile Pro Gly Asp Ile Ala Lys Glu Asp Ala Gly Asp
       155                 160                 165
   ggt act tat tcc aat tcc act att tct tct ggc act cct gtg gtg ttc      881
   Gly Thr Tyr Ser Asn Ser Thr Ile Ser Ser Gly Thr Pro Val Val Phe
   170                 175                 180                 185
   ccg gat cct act gag gct gca gcg ctg gtg gag gcg att aac aac gct      929
   Pro Asp Pro Thr Glu Ala Ala Ala Leu Val Glu Ala Ile Asn Asn Ala
                   190                 195                 200
   aag tct gtc act ttg ttc tgc ggt gcg ggc gtg aag aat gct cgc gcg      977
   Lys Ser Val Thr Leu Phe Cys Gly Ala Gly Val Lys Asn Ala Arg Ala
               205                 210                 215
   cag gtg ttg gag ttg gcg gag aag att aaa tca ccg atc ggg cat gcg      1025
   Gln Val Leu Glu Leu Ala Glu Lys Ile Lys Ser Pro Ile Gly His Ala
           220                 225                 230
   ctg ggt ggt aag cag tac atc cag cat gag aat ccg ttt gag gtc ggc      1073
   Leu Gly Gly Lys Gln Tyr Ile Gln His Glu Asn Pro Phe Glu Val Gly
       235                 240                 245
   atg tct ggc ctg ctt ggt tac ggc gcc tgc gtg gat gcg tcc aat gag      1121
   Met Ser Gly Leu Leu Gly Tyr Gly Ala Cys Val Asp Ala Ser Asn Glu
   250                 255                 260                 265
   gcg gat ctg ctg att cta ttg ggt acg gat ttc cct tat tct gat ttc      1169
   Ala Asp Leu Leu Ile Leu Leu Gly Thr Asp Phe Pro Tyr Ser Asp Phe
                       270                 275                 280
   ctt cct aaa gac aac gtt gcc cag gtg gat atc aac ggt gcg cac att    1217
   Leu Pro Lys Asp Asn Val Ala Gln Val Asp Ile Asn Gly Ala His Ile
               285                 290                 295
   ggt cga cgt acc acg gtg aag tat ccg gtg acc ggt gat gtt gct gca    1265
   Gly Arg Arg Thr Thr Val Lys Tyr Pro Val Thr Gly Asp Val Ala Ala
           300                 305                 310
   aca atc gaa aat att ttg cct cat gtg aag gaa aaa aca gat cgt tcc    1313
   Thr Ile Glu Asn Ile Leu Pro His Val Lys Glu Lys Thr Asp Arg Ser
       315                 320                 325
   ttc ctt gat cgg atg ctc aag gca cac gag cgt aag ttg agc tcg gtg    1361
   Phe Leu Asp Arg Met Leu Lys Ala His Glu Arg Lys Leu Ser Ser Val
   330                 335                 340                 345
   gta gag acg tac aca cat aac gtc gag aag cat gtg cct att cac cct    1409
   Val Glu Thr Tyr Thr His Asn Val Glu Lys His Val Pro Ile His Pro
                   350                 355                 360
   gaa tac gtt gcc tct att ttg aac gag ctg gcg gat aag gat gcg gtg    1457
   Glu Tyr Val Ala Ser Ile Leu Asn Glu Leu Ala Asp Lys Asp Ala Val
               365                 370                 375
   ttt act gtg gat acc ggc atg tgc aat gtg tgg cat gcg agg tac atc    1505
   Phe Thr Val Asp Thr Gly Met Cys Asn Val Trp His Ala Arg Tyr Ile
           380                 385                 390
   gag aat ccg gag gga acg cgc gac ttt gtg ggt tca ttc cgc cac ggc    1553
   Glu Asn Pro Glu Gly Thr Arg Asp Phe Val Gly Ser Phe Arg His Gly
       395                 400                 405
   acg atg gct aat gcg ttg cct cat gcg att ggt gcg caa agt gtt gat    1601
   Thr Met Ala Asn Ala Leu Pro His Ala Ile Gly Ala Gln Ser Val Asp
   410                 415                 420                 425
   cga aac cgc cag gtg atc gcg atg tgt ggc gat ggt ggt ttg ggc atg    1649
   Arg Asn Arg Gln Val Ile Ala Met Cys Gly Asp Gly Gly Leu Gly Met
                   430                 435                 440
   ctg ctg ggt gag ctt ctg acc gtt aag ctg cac caa ctt ccg ctg aag    1697
   Leu Leu Gly Glu Leu Leu Thr Val Lys Leu His Gln Leu Pro Leu Lys
               445                 450                 455
   gct gtg gtg ttt aac aac agt tct ttg ggc atg gtg aag ttg gag atg    1745
   Ala Val Val Phe Asn Asn Ser Ser Leu Gly Met Val Lys Leu Glu Met
           460                 465                 470
   ctc gtg gag gga cag cca gaa ttt ggt act gac cat gag gaa gtg aat    1793
   Leu Val Glu Gly Gln Pro Glu Phe Gly Thr Asp His Glu Glu Val Asn
       475                 480                 485
   ttc gca gag att gcg gcg gct gcg ggt atc aaa tcg gta cgc atc acc    1841
   Phe Ala Glu Ile Ala Ala Ala Ala Gly Ile Lys Ser Val Arg Ile Thr
   490                 495                 500                 505
   gat ccg aag aaa gtt cgc gag cag cta gct gag gca ttg gca tat cct    1889
   Asp Pro Lys Lys Val Arg Glu Gln Leu Ala Glu Ala Leu Ala Tyr Pro
                   510                 515                 520
   gga cct gta ctg atc gat atc gtc acg gat cct aat gcg ctg tcg atc    1937
   Gly Pro Val Leu Ile Asp Ile Val Thr Asp Pro Asn Ala Leu Ser Ile
               525                 530                 535
   cca cca acc atc acg tgg gaa cag gtc atg gga ttc agc aag gcg gcc    1985
   Pro Pro Thr Ile Thr Trp Glu Gln Val Met Gly Phe Ser Lys Ala Ala
           540                 545                 550
   acc cga acc gtc ttt ggt gga gga gta gga gcg atg atc gat ctg gcc    2033
   Thr Arg Thr Val Phe Gly Gly Gly Val Gly Ala Met Ile Asp Leu Ala
       555                 560                 565
   cgt tcg aac ata agg aat att cct act cca tgatgattga tacacctgct      2083
   Arg Ser Asn Ile Arg Asn Ile Pro Thr Pro
   570                 575
   gttctcattg accgcgagcg cttaactgcc aacatttcca ggatggcagc tcacgccggt 2143
   gcccatgaga ttgccct                                                 2160
<210> 5
   <211> 579
   <212> PRT
   <213> Corynebacterium glutamicum
<400> 5
   Met Ala His Ser Tyr Ala Glu Gln Leu Ile Asp Thr Leu Glu Ala Gln
   1 5 10 15
   Gly Val Lys Arg Ile Tyr Gly Leu Val Gly Asp Ser Leu Asn Pro Ile
   20 25 30
   Val Asp Ala Val Arg Gln Ser Asp Ile Glu Trp Val His Val Arg Asn
   35 40 45
   Glu Glu Ala Ala Ala Phe Ala Ala Gly Ala Glu Ser Leu Ile Thr Gly
   50 55 60
   Glu Leu Ala Val Cys Ala Ala Ser Cys Gly Pro Gly Asn Thr His Leu
   65 70 75 80
   Ile Gln Gly Leu Tyr Asp Ser His Arg Asn Gly Ala Lys Val Leu Ala
   85 90 95
   Ile Ala Ser His Ile Pro Ser Ala Gln Ile Gly Ser Thr Phe Phe Gln
   100 105 110
   Glu Thr His Pro Glu Ile Leu Phe Lys Glu Cys Ser Gly Tyr Cys Glu
   115 120 125
   Met Val Asn Gly Gly Glu Gln Gly Glu Arg Ile Leu His His Ala Ile
   130 135 140
   Gln Ser Thr Met Ala Gly Lys Gly Val Ser Val Val Val Ile Pro Gly
   145 150 155 160
   Asp Ile Ala Lys Glu Asp Ala Gly Asp Gly Thr Tyr Ser Asn Ser Thr
   165 170 175
   Ile Ser Ser Gly Thr Pro Val Val Phe Pro Asp Pro Thr Glu Ala Ala
   180 185 190
   Ala Leu Val Glu Ala Ile Asn Asn Ala Lys Ser Val Thr Leu Phe Cys
           195                 200                 205
   Gly Ala Gly Val Lys Asn Ala Arg Ala Gln Val Leu Glu Leu Ala Glu
       210                 215                 220
   Lys Ile Lys Ser Pro Ile Gly His Ala Leu Gly Gly Lys Gln Tyr Ile
   225                 230                 235                 240
   Gln His Glu Asn Pro Phe Glu Val Gly Met Ser Gly Leu Leu Gly Tyr
                   245                 250                 255
   Gly Ala Cys Val Asp Ala Ser Asn Glu Ala Asp Leu Leu Ile Leu Leu
               260                 265                 270
   Gly Thr Asp Phe Pro Tyr Ser Asp Phe Leu Pro Lys Asp Asn Val Ala
           275                 280                 285
   Gln Val Asp Ile Asn Gly Ala His Ile Gly Arg Arg Thr Thr Val Lys
       290                 295                 300
   Tyr Pro Val Thr Gly Asp Val Ala Ala Thr Ile Glu Asn Ile Leu Pro
   305                 310                 315                 320
   His Val Lys Glu Lys Thr Asp Arg Ser Phe Leu Asp Arg Met Leu Lys
                   325                 330                 335
   Ala His Glu Arg Lys Leu Ser Ser Val Val Glu Thr Tyr Thr His Asn
               340                 345                 350
   Val Glu Lys His Val Pro Ile His Pro Glu Tyr Val Ala Ser Ile Leu
           355                 360                 365
   Asn Glu Leu Ala Asp Lys Asp Ala Val Phe Thr Val Asp Thr Gly Met
       370                 375                 380
   Cys Asn Val Trp His Ala Arg Tyr Ile Glu Asn Pro Glu Gly Thr Arg
   385                 390                 395                 400
   Asp Phe Val Gly Ser Phe Arg His Gly Thr Met Ala Asn Ala Leu Pro
                   405                 410                 415
   His Ala Ile Gly Ala Gln Ser Val Asp Arg Asn Arg Gln Val Ile Ala
               420                 425                 430
   Met Cys Gly Asp Gly Gly Leu Gly Met Leu Leu Gly Glu Leu Leu Thr
           435                 440                 445
   Val Lys Leu His Gln Leu Pro Leu Lys Ala Val Val Phe Asn Asn Ser
       450                 455                 460
   Ser Leu Gly Met Val Lys Leu Glu Met Leu Val Glu Gly Gln Pro Glu
   465                 470                 475                 480
   Phe Gly Thr Asp His Glu Glu Val Asn Phe Ala Glu Ile Ala Ala Ala
                   485                 490                 495
   Ala Gly Ile Lys Ser Val Arg Ile Thr Asp Pro Lys Lys Val Arg Glu
               500                 505                 510
   Gln Leu Ala Glu Ala Leu Ala Tyr Pro Gly Pro Val Leu Ile Asp Ile
           515                 520                 525
   Val Thr Asp Pro Asn Ala Leu Ser Ile Pro Pro Thr Ile Thr Trp Glu
   530                 535                 540
   Gln Val Met Gly Phe Ser Lys Ala Ala Thr Arg Thr Val Phe Gly Gly
   545                 550                 555                 560
   Gly Val Gly Ala Met Ile Asp Leu Ala Arg Ser Asn Ile Arg Asn Ile
                   565                 570                 575
   Pro Thr Pro
<210> 6
   <211> 875
   <212> DNA
   <213> Corynebacterium glutamicum
<400> 6
   tgcgagatgg tgaatggtgg tgagcagggt gaacgcattt tgcatcacgc gattcagtcc 60
   accatggcgg gtaaaggtgt gtcggtggta gtgattcctg gtgatatcgc taaggaagac 120
   gcaggtgacg gtacttattc caattccact atttcttctg gcactcctgt ggtgttcccg 180
   gatcctactg aggctgcagc gctggtggag gcgattaaca acgctaagtc tgtcactttg 240
   ttctgcggtg cgggcgtgaa gaatgctcgc gcgcaggtgt tggagttggc ggagaagatt 300
   aaatcaccga tcgggcatgc gctgggtggt aagcagtaca tccagcatga gaatccgttt 360
   gaggtcggca tgtctggcct gcttggttac ggcgcctgcg tggatgcgtc caatgaggcg 420
   gatctgctga ttctattggg tacggatttc ccttattctg atttccttcc taaagacaac 480
   gttgcccagg tggatatcaa cggtgcgcac attggtcgac gtaccacggt gaagtatccg 540
   gtgaccggtg atgttgctgc aacaatcgaa aatattttgc ctcatgtgaa ggaaaaaaca 600
   gatcgttcct tccttgatcg gatgctcaag gcacacgagc gtaagttgag ctcggtggta 660
   gagacgtaca cacataacgt cgagaagcat gtgcctattc accctgaata cgttgcctct 720
   attttgaacg agctggcgga taaggatgcg gtgtttactg tggataccgg catgtgcaat 780
   gtgtggcatg cgaggtacat cgagaatccg gagggaacgc gcgactttgt gggttcattc 840
   cgccacggca cgatggctaa tgcgttgcct catgc                            875